Europäisches Patentamt

(19) European Patent Office

Office européen des brevets

(11) Numéro de publication: **0 235 474**
**A1**

(12) **DEMANDE DE BREVET EUROPEEN**

(21) Numéro de dépôt: **86402711.5**

(22) Date de dépôt: **21.11.86**

(51) Int. Cl.⁴: **C 12 N 15/00,** C 07 K 15/00, A 61 K 39/10

(30) Priorité: **22.11.85 FR 8517359**

(43) Date de publication de la demande: **09.09.87**
**Bulletin 87/37**

(84) Etats contractants désignés: **ES GR**

(71) Demandeur: **INSTITUT PASTEUR, 25-28, rue du Docteur Roux, F-75724 Paris Cédex 15 (FR)**

(72) Inventeur: **Ulmann, Agnès, 3, rue Paul Dupuy, F-75016 Paris (FR)**
Inventeur: **Mattei, Denise, 142, rue du Théatre, F-75015 Paris (FR)**
Inventeur: **Mercereau-Puijalon, Odile, 11, bld.Voltaire, F-92130 Issy Les Moulineaux (FR)**
Inventeur: **Alonso, Jean-Michel, 51, rue de Cornouailles, F-78180 Montigny-le-Bretonneux (FR)**
Inventeur: **Shozo Ozaki, Luiz, 63, rue Blomet, F-75015 Paris (FR)**
Inventeur: **Pichot, Frédérique, 6, rue des Fossés-St-Denis, F-92100 Boulogne (FR)**

(74) Mandataire: **Gutmann, Ernest et al, S.C. Ernest Gutmann - Yves Plasseraud 67, boulevard Haussmann, F-75008 Paris (FR)**

(54) **Antigènes purifiés ayant des propriétés vaccinantes contre B. pertussis, moyens notamment ADNs recombinants pour les produire et compositions de vaccins les contenant.**

㊿ L'invention concerne des polypeptides susceptibles de former des principes actifs de vaccins purifiés contre la coqueluche. Ils sont produits par génie génétique. Ils sont reconnus par des anticorps anti-FHA, anti-LPF et anti-CYA. L'invention concerne également les séquences d'ADN codant pour lesdits polypeptides, ainsi que les compositions immunogènes associant lesdits polypeptides avec des véhicules pharmaceutiquement acceptables.

EP 0 235 474 A1

1

<u>Antigènes purifiés ayant des propriétés vaccinantes contre B. pertussis, moyens notamment ADNs recombinants pour les produire et compositions de vaccins les contenant</u> ·

L'invention concerne des polypeptides susceptibles de former des principes actifs de vaccins purifiés contre la coqueluche. Ces vaccins sont dirigés contre des antigènes distincts de l'agent pathogène. L'invention concerne plus particulièrement les associations qui peuvent être formées entre ces principes vaccinants. Ceux-ci sont susceptibles d'être obtenus par les techniques du génie génétique.

L'invention concerne donc aussi les séquences d'ADN codant pour les susdits polypeptides, ainsi que les vecteurs qui les contiennent.

La coqueluche est une infection bactérienne, due à <u>Bordetella pertussis</u>, qui provoque une trachéo-bronchite non septique, parfois mortelle chez l'enfant de moins de six mois, représentant la classe d'âge la plus exposée sinon exclusive.

Cette infection, connue depuis plus de cinq siècles, sévit dans tous les pays et n'est contrôlée que par la vaccination préventive, systématiquement instaurée dans les pays développés depuis 1945. Une fois la maladie installée dans sa phase d'expression clinique, aucun recours thérapeutique spécifique n'est possible, les soins sont alors purement symptomatiques et visent à prévenir les phénomènes anoxiques et les complications infectieuses secondaires qui sont les causes de la mortalité.

Les antibiotiques actifs de <u>B. pertussis</u> n'influencent pas l'évolution de la coqueluche dont les médiateurs de pathogénicité sont extra-bactériens.

La vaccination est, par contre, le moyen efficace de contenir les flambées épidémiques. La preuve indirecte de l'efficacité de la vaccination est apportée par les exemples récents du Japon et de la Grande-Bretagne où la levée de ce mode de prévention a été santionnee par de

violentes épidémies avec des cas mortels.

Les vaccins actuellement employés dans le monde entier sont majoritairement composés de suspensions de B. pertussis, simplement lavées par centrifugation puis tuées par. chauffage à 56°C ou quelquefois par adjonction d'un antiseptique. Le chauffage à 56°C a pour but essentiel d'inactiver la toxine dermonécrotique libérée par les bactéries lysées.

On reconnaît actuellement deux antigènes immuno-protecteurs chez B. pertussis :

L'hémagglutinine filamenteuse (FHA), associée aux fimbriae (protéine de PM > 150 000), thermodénaturée à 56°C, dénuée d'effet pathogène mais facteur dominant dans l'adhésion aux cellules épithéliales respiratoires.

Un autre antigène est connu sous les appellations "facteur promoteur d'une leucocytose" (LPF selon la désignation qui sera utilisée dans la suite de cette description) ou encore toxine pertussique (Ptx) ou facteur sensibilisant à l'histamine (HSF) ou protéine activant les ilots $\beta$ du pancrés endocine (IAP). Ces différentes appellations évoquent les multiples effets biologiques de cette protéine pentamérique (PM ± 95 000) qui sont dus en fait à la sous-unité $\alpha$ qui possède une fonction ADPribosyl-transférase, agissant sur la sous-unité inhibitrice du site de régulation de l'adényle cyclase des cellules eucaryotes, en bloquant ainsi la régulation inhibitrice de la synthèse d'AMP cyclique eucaryote. La fonction d'attachement de cette protéine serait portée par d'autres structures non encore identifiées de la molécule.

L'intérêt de ces deux antigènes excrétés par B. pertussis en culture a conduit à l'exploitation de leur purification pour la mise au point du premier vaccin coquelucheux acellulaire qui est actuellement en exploitation à l'échelle industrielle au Japon.

Outre ces deux antigènes pour lesquels des études biochimiques, immunochimiques et physiopathologiques étoffées ont apporté de solides arguments en faveur de leurs qualités immunogènes. Des études récentes des inventeurs les ont conduits à la présomption plus que sérieuse qu'un troisième produit, synthétisé et excrété par B. pertussis : l'adényle cyclase (CYA) est susceptible d'intervenir comme un immunogène jouant un rôle important dans l'immunoprotection anticoquelucheuse.

D'autres antigènes, encore peu étudiés peuvent se révéler comme participant également à l'immunogénicité de B. pertussis : il s'agit du lipopolysaccharide (LPS) dont les effets biologiques sont ceux de toute endotoxine bactérienne, de la toxine dermonécrotique qui est une protéine cystoplasmique, responsable de nécroses cutanées, et de la cytotoxine trachéale dont l'existence et le rôle spécifique sur l'épithélium sont suspectés, d'après les expériences in vitro de prélèvement de trachée de hamster.

Bien que les trois immunogènes FHA, Ptx et CYA, soient extracellulaires, leur purification biochimique pose encore de nombreux problèmes :
- copurifications liées probablement à des mécanismes de relargage à partir de sites structurellement associés sur l'enveloppe cellulaire ;
- rendements de synthèse faibles et difficilement reproductibles à l'échelle industrielle et surtout
- variations phénotypiques de B. pertussis. en cultures en milieux synthétiques, liées vraisemblablement à la modulation de la synthèse notamment du LPF et de CYA par des constituants du milieu identifiés (nicotinamide, glutathion, ions $Mg^{2+}$) ou les conditions de pression d'oxygène et de pH.

L'invention a donc pour but de fournir des polypeptides susceptibles d'être obtenus avec un haut degré de purification et de reproductibilité par des techniques du génie génétique. Ces polypeptides sont obtenus par expression dans un hôte approprié de séquences d'acides nucléiques, qui ont été obtenues d'une banque de séquences

4

nucléotidiques elles-mêmes préalablement obtenues à partir de l'ADN génomique de B. pertussis 8144 (selon la référence de l'Institut Pasteur de Paris) sérotype 1.3.

Construction de la banque de B. pertussis

30 µg de DNA de B. pertussis ont été incubés à 15°C pendant 5 ou 10 minutes dans un volume final de 450 µl en tampon 50mM Tris HCl 7,5, MnCl$_2$ 10 mM, sérum albumine bovine (SIGMA) 20 µg/ml, en présence de 330 µg/ml de déoxyribonucléase pancréatique (DNAase I Boehringer Mannheim). On a ainsi obtenu des fragments ayant des tailles comprises entre environ 1 et environ 8 kb. La réaction a été arrêtée par addition d'EDTA 5mM final. Le DNA a été extrait par un volume égal du mélange phénol/chloroforme/alcool isoamylique (25V/24V/1V), puis précipité par l'éthanol.

5 µg de DNA ainsi obtenu ont été incubés pendant une heure à 15°C dans un volume de 60 µl en tampon 40 mM Tris HCl pH 7.9, 10 mM sulfate d'ammonium, 10 mM β-mercaptoéthanol, 0,5 mM EDTA, 200 µM de dATP, dCTP, dGTP, dTTP, 100 µM β-NAD) en présence de 25 U de T4 DNA-polymérase (P.L. Biochemicals) et de 60 U de E. coli DNA ligase (New England Biolabs).

Après extraction au phénol et précipitation par l'éthanol comme ci-dessus, le DNA a été incubé dans un volume de 22,5 µl avec 0,25 µg de linkers EcoRI phosphorylés (New England Biolabs) en présence de 1000 U de T4 DNA ligase (New England Biolabs) dans le tampon adéquat (66 mM Tris HCl pH 7,5, 5 mM MgCl$_2$, 5 mM DTT et 0,5 mM ATP). L'incubation a été effectuée à 4°C pendant 4 heures. 1000 U de T4 DNA ligase (New England Biolabs) ont été rajoutées et l'incubation poursuivie 16 heures à 4°C. Le mélange a été soumis à deux cycles successifs de congélation à -80°C, suivie de décongélation. Le volume a été amené à 50 µl avec de l'eau.

Le DNA a été ensuite incubé dans un volume de 250 µl en présence de 50 unités d'EcoRI (New England Biolabs)

5

pendant 3 heures à 37°C dans le tampon recommandé par le fournisseur. Après incubation 10 minutes à 60°C, le DNA a été extrait au phénol comme ci-dessus. Après addition d'acétate d'ammonium 2,5 M final, le DNA a été précipité par 2 volumes d'éthanol pendant 16 heures à -20°C.

500 ng ou 1 µg de DNA digéré 5 minutes, ou 500 ng ou 1 µg de DNA digéré 10 minutes ont été ligaturés indépendamment (dans le tampon de ligation recommandé par Promega Biotec) à 250 ng de DNA du vecteur λgt11 (Proto-clone TM GT System, Promega Biotec) dans un volume de 5 µl en présence de 200 unités de T4 DNA polymérase (New England Biolabs). La réaction a été effectuée à 4°C pendant 16 heures.

Les produits obtenus ont été encapsidés dans des capsides du bactériophage λ. Les extraits d'encapsidation ont été préparés selon le protocole décrit par Maniatis et Coll. (Molecular cloning. A laboratory manual. Cold Spring Harbour, p. 264-267, 1982).

En bref, l'encapsidation est effectuée comme suit: 1 µl de DNA ligaturé (50 ng de vecteur) sont ajoutés à 7 µl de tampon A à 4°C (20 mM Tris HCl pH 8 ;3 mM $MgCl_2$, 0,05 % β-mercaptoéthanol, 1 mM EDTA), puis on ajoute 1 µl de tampon ML (6 mM Tris pH 7,4, 18 mM $MgCl_2$, 30 mM spermidine, 30 mM β-mercaptoéthanol, 15 mM putrescine). 6 µl d'extrait d'encapsidation SE décongelé à 4°C sont ajoutés puis 10 µl d'extrait d'encapsidation FTL décongelé à 4°C. Le mélange est incubé 1 heure à 25°C, puis dilué à 500 µl avec du tampon Tris 10 mM pH 7.4, 10 mM $MgCl_2$, et étalé sur des bactéries indicatrices.

Les extraits FTL et SE sont des extraits respectivement des souches bactériennes BHB 2688 et BHB 2690 thermo-induites. Le génotype de souches est décrit dans Maniatis et Coll. op.cit p. 504. Dans le cas présent, les extraits obtenus permettaient d'obtenir environ $10^9$ bactériophages par µg de DNA de λCI857.

Après l'encapsidation, les phages ont été criblés

afin de déterminer le pourcentage des phages recombinants.

Environ 60 % des phages recombinants ont été obtenus (d'après un nombre de plages de lyse blanches par rapport aux bleues sur boîtes indicatrices contenant IPTG + X-gal).

Le rendement final était de 300 000 phages recombinants par microgramme de vecteur utilisé.

La banque ainsi obtenue contenant environ 300 fois le génome de B. pertussis a été criblée en utilisant un mélange de sérums humains de malades ayant contracté la coqueluche.

Environ 30 % des plages de lyse ont donné une réponse positive.

Le criblage avec des antisérums spécifiques dirigés contre les trois antigènes purifiés (LPF, FHA et CYA) a donné des résultats positifs ; environ 0,3 à 0,5 % des phages recombinants contiennent les séquences individuelles représentant ces antigènes.

Ce criblage a été effectué à l'aide des anticorps préalablement formés chez le lapinou la souris contre le LPF, la FHA et la CYA (plus particulièrement contre la protéine de poids moléculaire d'environ 67 000, obtenue par le groupe auquel les inventeurs de la présente demande appartiennent, à partir de cette CYA, respectivement et, d'autre part, avec des anticorps contenus :
- dans des sérums humains hyperimmunisés par vaccin coquelucheux,
- dans des sérums d'enfants malades non vaccinés.

Pour le criblage, la technique et la souche RY1090 utilisées ont été décrites par R. YOUNG et al. (Proc. Natl. Acad. Sci. USDA, vol. 82 (1985), 2583-2587.

La souche RY1090, cultivée jusqu'à saturation en milieu LB contenant du maltose (0,02 %) a été centrifugée et reprise dans $MgSO_4$ 10 mM. Environ $10^4$ phages de la banque ont été incubés, avec environ $2.10^8$ bactéries pendant 15 minutes à 37°C, puis étalés sur boîte TRYPTONE.

7

Après 3 heures d'incubation à 42°C, les boîtes ont été amenées à 37°C et un filtre de nitrocellulose (SCHLEICHER & SCHULL) imprégné dans isopropoyl-β-D-thiogalactoside (IPTG) 10 mM, a été posé dessus.

Après 3 heures d'incubation à 37°C, le premier filtre est retiré puis, pour avoir une deuxième empreinte, un deuxième filtre, également imprégné par IPTG, a été posé dessus pour 3 heures supplémentaires.

Les filtres ont été trempés dans un tampon TBS (Tris 50 mM NaCl 150 mM).

On expose ci-après les principales étapes du procédé qui a été utilisé pour la détection des filtres par les anticorps :

- Incubation des filtres dans un tampon TBS contenant 5 % de lait écrémé (marque REGILAIT) et 0,01 % d'azide de sodium (TBS lait) pendant 1 heure à température ambiante ;
- Incubation avec l'immunsérum de TBS lait, 1 heure à 37°C;
- Lavage 3 fois en TBS lait

    1 lavage TBS lait + 0,1 % NP-40,

    1 lavage TBS lait.

- Incubation avec protéine A marquée à l'$^{125}$I (0,5 micro-Curie par filtre), 1 heure à température ambiante ;
- Lavage 3 fois TBS lait ;

    1 lavage TBS lait, NP-40 ;

    1 lavage TBS lait,

    2 lavages TBS ;

- Séchage des filtres - Autoradiographie à 80°C avec écran.

A l'endroit des signaux positifs, l'agar contenant des particules de phages a été enlevé, incubé 1 heure dans un tampon phosphate 100 mM MgSO$_4$ 10 mM à 37°C. Les phages donnant des signaux positifs ont été repurifiés, le nombre de fois nécessaires pour obtenir autant de plages de lyse par boîte que de signaux positifs en autoradiographie.

8

La révélation des plages de lyse donnant des signaux potifis a été, dans certains cas, réalisée par des anticorps anti-Ig lapin-souris ou homme marqués à la peroxydase (BIOSYS). Ceci a été surtout nécessaire lors de l'utilisation de sérums de souris.

Les plages de lyse donnant des signaux positifs avec des sérums monospécifiques, dirigés contre la CYA (sérum de lapin), contre le FHA (sérum de souris) et contre le LPH (sérums de souris ou de lapin) ont été lysogénisés dans la souche RY1090, par des techniques classiques. Les lysogènes ont été définis comme ayant une croissance à 30°C et pas de croissance à 42°C. Les clones lysogènes s'appellent respectivement :

RY1090 λgt11 lys CYA

RY1090 λgt11 lys LPF

RY1090 λgt11 lys FHA.

Ces clones ont été déposés à la Collection Nationale des Cultures de Micro-organismes de l'Institut Pasteur (C.N.C.M.) le 29 juillet 1985, sous les n° I-474, I-475 et I-476.

Lorsque les phages de lyse donnant des signaux positifs avec les sérums monospécifiques (mentionnés ci-dessus) ont été criblés par les sérums humains hyperimmunisés ou provenant de malades, ils ont donné, dans la majorité des cas, également des signaux positifs.

Les lysogènes obtenus dans la souche RY1090 permettent l'obtention et l'amplification des phAges recombinants portant les insertions correspondantes aux antigènes LPF, FHA et CYA. Ils permettent également l'obtention, selon des techniques classiques, de l'ADN correspondant lequel, par marquage adéquat, peut servir de sonde spécifique.

Afin d'obtenir les protéines-fusion contenues dans les phages recombinants, la lysogénisation est effectuée dans la souche RY1089 portant la mutation Hf1 (décrite par YOUNG et al.).

9

L'obtention des cultures bactériennes contenant les protéines-fusion recherchées est obtenue par la culture de cette souche à 30°C, suivie par une induction de 20 minutes à 42°C, puis par une induction de l'IPTG 10 mM, enfin par une incubation pendant 1 heure à 37°C.

Les protéines peuvent ensuite être purifiées par des techniques classiques.

La potentialité vaccinante et protectrice des protéines-fusion est mesurable par le test de Kendricke, préconisé par l'Organisation Mondiale de la Santé (OMS), lequel permet l'étude de la pathogénicité et de l'immunogénicité de B. pertussis observée à l'occasion de l'infection expérimentale de la souris. Il consiste en l'estimation de la dose léthale : 50 % de la souche de référence internationale (OMS) 18323 (sérotypes 1, 2, 3, 4, 5, 7) pour des souris immunisées ou non. La mesure de tout immunogène à potentialité "vaccinante" est effectuée par comparaison avec un vaccin d'efficacité connue.

L'invention concerne donc plus particulièrement l'utilisation de la CYA ou de protéines purifiées qui peuvent être obtenues à partir de celles-ci pour la constitution de principes vaccinants contre la coqueluche.

Un vaccin préféré selon l'invention contient une association d'antigènes ayant les caractéristiques de la FHA, du LPF et de la CYA. On peut ainsi bénéficier des effets caractéristiques simultanés de ces trois types d'antigènes.

Des données expérimentales basées sur la séroneutralisation de l'infection par B. pertussis de fibroblastes 3T3, montrent que les anticorps anti-FHA protègent contre l'adhésion des bactéries aux membranes cellulaires, mais ne semblent pas neutraliser les effets cytotoxiques, que les anticorps anti-LPH, outre un faible pouvoir inhibiteur de l'adhésion, protègent les cellules contre les phénomènes cytotoxiques et que cet effet est sensiblement potentialisé par les anticorps anti-CYA.

10

Un tel vaccin paraît capable d'immuniser contre chacun des principaux déterminants impliqués dans le pouvoir pathogène de B. pertussis : le FHA en tant que responsable majeur de l'adhésion et de la prolifération in situ de l'inoculum bactérien, et le LPH et CYA en tant que cytotoxines capables d'agir indépendamment du FHA.

L'invention concerne au même titre les compositions de vaccin associant les produits d'expression des séquences d'insertion contenues dans les trois phages sus-indiqués (ou tout produit semblable doué de propriétés immunogènes semblables) et reconnues par des anticorps anti-FHA, anti-LPF et anti-CYA.

Naturellement, l'invention concerne également les compositions de vaccin contenant des doses efficaces des principes actifs de vaccins susmentionnés, en association avec tout véhicule pharmaceutique approprié au mode d'administration choisi, par exemple par la voie orale, parentérale, nasale ou rectale.

L'invention concerne enfin les séquences d'acides nucléiques d'insertion elles-mêmes ou toute séquence codant pour des polypeptides semblables. Font notamment partie de l'invention les séquences d'acides nucléiques qui sont susceptibles de s'hybrider avec les séquences d'insertion contenues dans les vecteurs qui ont été déposés à la C.N.C.M.

## REVENDICATIONS

1 - Acide nucléique recombinant contenant une séquence d'insertion issue du génome de B. pertussis, contenue dans le phage lui-même déposé à la C.N.C.M. sous le n° I-474.

2 - Acide nucléique recombinant contenant une séquence d'insertion issue du génome de B. pertussis, contenue dans le phage lui-même déposé à la C.N.C.M. sous le n° I-475.

3 - Acide nucléique recombinant contenant une séquence d'insertion issue du génome de B. pertussis, contenue dans le phage lui-même déposé à la C.N.C.M. sous le n° I-476.

4 - Le produit d'expression, dans un hôte approprié, de la séquence d'insertion de l'acide nucléique selon la revendication 1.

5 - Le produit d'expression, dans un hôte approprié, de la séquence d'insertion de l'acide nucléique selon la revendication 2.

6 - Le produit d'expression, dans un hôte approprié, de la séquence d'insertion de l'acide nucléique selon la revendication 3.

7 - Composition immunogène contenant le produit d'expression selon l'une quelconque des revendications 4, 5 et 6.

8 - Composition immunogène selon la revendication 7, caracérisée en ce qu'elle contient l'ensemble des produits d'expression tels que définis dans les revendications 4, 5 et 6.

9 - Composition vaccinante contre B. pertussis, caractérisée en ce qu'elle contient les principes actifs de la composition immunogène selon la revendication 7 ou la revendication 8, en association avec un véhicule pharmaceutiquement acceptable.

Office européen
des brevets

**RAPPORT DE RECHERCHE EUROPEENNE**

Numéro de la demande

EP 86 40 2711

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | PROCEEDINGS OF THE NATIONAL ACADEMY OF SCIENCES, vol. 82, mai 1985, pages 2583-2587; R.A. YOUNG et al.: "Dissection of Mycobacterium tuberculosis antigens using recombinant DNA" * En entier * | 1-9 | C 12 N 15/00 C 07 K 15/00 A 61 K 39/10 |
| Y | THE JOURNAL OF IMMUNOLOGY, vol. 130, no. 6, juin 1983, pages 2767-2774, The American Association of Immunologists, US; M. SUGIMOTO et al.: "Effect of bordetella pertussis leukocytosis (lymphocytosis)-promoting factor (LPF) on the physical lymphoepithelial-cell association studied with the use of an in vitro model of mouse thymus" * Abrégé * | 2,5,7-9 | |
| Y | ACTA PATHOLOGICA ET MICROBIOLOGICA SCANDINAVICA, section C, vol. 92, 1984, pages 279-284; J.C. SELMER et al.: "Purification and partial characterization of filamentous haemagglutinin from Bordetella pertussis using monoclonal antibodies" * En entier * ---    -/- | 3,6-9 | **DOMAINES TECHNIQUES RECHERCHES (Int. Cl.4)** A 61 K C 12 N C 12 P |

Le présent rapport de recherche a été établi pour toutes les revendications

| Lieu de la recherche | Date d'achèvement de la recherche | Examinateur |
|---|---|---|
| LA HAYE | 09-04-1987 | CUPIDO M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03 82

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl.4) |
|---|---|---|---|
| Y | INFECTION AND IMMUNITY, vol. 50, no. 1, octobre 1985, pages 199-206, American Society for Microbiology; P. NOVOTNY et al.: "Adenylate cyclase activity of a 68,000-molecular-weight protein isolated from the outer membrane of Bordetella bronchiseptica" * Abrégé * | 1,4,7-9 | |
| A | JOURNAL OF BACTERIOLOGY, vol. 159, no. 2, août 1984, pages 780-782, American Society for Microbiology; F. SHARECK et al.: "Cloning of Bordetella pertussis outer membrane proteins in Escherichia coli" * En entier * | 1-9 | |

DOMAINES TECHNIQUES
RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 09-04-1987 | CUPIDO M. |